# EUROPEAN PATENT SPECIFICATION

(11) **EP 2 041 289 B1**
(45) Date of publication and mention of the grant of the patent: **17.11.2010**
(21) Application number: 07787193.7
(22) Date of filing: 06.07.2007
(51) Int. Cl.: C12N 15/82

(54) **RESISTANCE TO POWDERY MILDEW AND ABSENCE OF NECROSIS IN CUCUMIS SATIVUS**
RESISTENZ GEGEN ECHTEN MEHLTAU UND FEHLEN VON NEKROSE BEI CUCUMIS SATIVUS
RÉSISTANCE AU MILDIOU PULVÉRULENT ET ABSENCE DE NÉCROSE DANS CUCUMIS SATIVUS

(30) Priority: 07.07.2006 WO PCT/EP2006/064033
(43) Date of publication of application: 01.04.2009
(73) Proprietor: Enza Zaden Beheer B.V., 1602 DB Enkhuizen (NL)
(72) Inventor: MAZEREEUW, Jacob Pieter, NL-1602 DB Enkhuizen (NL); SCHOENMAKERS, Marinus Cornelius Maria, NL-1602 DB Enkhuizen (NL); VAN KAMPEN, Brigitta Veronica, NL-1602 DB Enkhuizen (NL); LAMBALK, Johannes Jacobus Maria, NL-1602 DB Enkhuizen (NL)
(74) Representative: van Kooij, Adriaan
(86) International application number: PCT/EP2007/056911
(87) International publication number: WO 2008/003783

(56) References cited:
- EP-A- 1 188 833
- EP-A- 1 433 378
- EP-A- 1 782 685
- SAKATA Y ET AL: "QTL analysis of powdery mildew resistance in cucumber (Cucumis sativus L.)" THEORETICAL AND APPLIED GENETICS, SPRINGER, BERLIN, DE, vol. 112, no. 2, 21 October 2005 (2005-10-21), pages 243-250, XP002374221 ISSN: 0040-5752
- MORISHITA M ET AL: "POWDERY MILDEW RESISTANCE IN CUCUMBER" JARQ. JAPAN AGRICULTURAL RESEARCH QUARTERLY, TOKYO, JP, vol. 37, no. 1, January 2003 (2003-01), pages 7-14, XP009063997 ISSN: 0021-3551
- XIAO S ET AL: "Enhanced transcription of the arabidopsis disease resistance genes RPW8.1 and RPW8.2 via a salicylic acid-dependent amplification circuit is required for hypersensitive cell death" PLANT CELL, AMERICAN SOCIETY OF PLANT PHYSIOLOGISTS, ROCKVILLE, MD, US, vol. 15, January 2003 (2003-01), pages 33-45, XP002980550 ISSN: 1040-4651

## Description

The present invention relates to powdery mildew-resistant Cucumis sativus plants which are necrosis-free. In addition, the invention relates to a method for obtaining powdery mildew-resistant cucumber plants which are necrosis-free.

The cucumber plant (i.e. a plant of the botanical species Cucumis sativus) belongs to the gourd family of Cucurbitaceae, like melons and squash. The cucumbers are the edible fruits of the plant, which are cylindrical, green-skinned fruits, consisting of about 96% water. The cucumber plant, which has been cultivated since long, is an important horticultural crop worldwide. Cucumbers are commonly harvested in an unripe stadium and may be used for the pickling industry or the fresh market.

Powdery mildew is one of the main fungal diseases known in cucumber plants, both in the field and greenhouse. Powdery mildew can be caused by Sphaerotheca fuliginea (Schlecht. ex Fr.)(recently renamed: Podosphaera xanthii) and/or Erysiphe cichoracearum DC (ex Mérat emend. Salm)(recently renamed: Golovinomyces cichoracearum). In greenhouse cultivation powdery mildew is predominantly caused by the first species. The fungus occurs mainly on leaves, which are most susceptible 2 to 3 weeks after unfolding. However, in severely affected plants the fungus may also occur on the stem and even the fruits. Severely affected leaves can become dry and brittle, or can wither and die. Because of the infection, the fruits can be smaller in size, fewer in number, less able to be successfully stored, sun scalded, incompletely ripe, and have a poor flavour. It may also predispose plants to be more vulnerable to other pathogens. Eventually, the plant can die.

Until now, fungicide application and the use of varieties with some resistance to the fungus have been the major methods of disease control. Thus, a resistance against both fungi has been demonstrated in various commercial cultivars. It has been demonstrated that hypocotyl resistance is based on a recessive gene (s), while leaf resistance is controlled by the dominant leaf gene (R). Both genes are necessary for a high-level resistance at the whole plant level (Shanmugasundaram, et al., Phytopathology 61: 1218-1221, 1971).

Powdery mildew (PM)-resistant cultivars, however, generally suffer from necrosis under low-light conditions (i.e. conditions wherein the light exposure of the plants is such that less than 2000 J/cm² of energy is received by the plant = less than 286 J/cm² per day), in particular in combination with a high fruit load, i.e. at least one fully developed fruit in a harvestable stage per node. Such conditions often occur during autumn, winter and early spring, in particular in production areas in Northern European countries and Canada. The fact that resistance against powdery mildew is associated with necrosis of the plants severely limits the practical use of these powdery mildew resistant plants.

The symptoms of necrosis related to powdery mildew resistance in cucumber begin with a yellowing between the main veins of the leaves (chlorosis), eventually resulting in necrosis (i.e. death of the leaves). A positive correlation between mildew resistance and necrosis sensitivity has been demonstrated, which has led to the suggestion that both traits are genetically tightly linked or that necrosis is a pleiotropic effect of one or more of the resistance genes.

In EP 1 433 378 a breaking of the genetic linkage between powdery mildew resistance and leaf necrosis in one Cucumis sativus line (DC-1) has been described. However, the genetic control of the powdery mildew resistance related necrosis phenomenon has not yet been elucidated, and many cucumber producers still suffer from the occurrence of necrosis in powdery mildew resistant cucumber cultivars. As a consequence, cucumber production still involves the use of fungicides for crop protection to control the infection with powdery mildew, which not only increases the costs involved but also is undesirable in view of a healthy environment.

In order to reduce the use of fungicides it thus is essential to provide plants, or to find new methods for providing plants, that are both resistant to powdery mildew and are necrosis-free.

The object of the invention is to provide Cucumis sativus plants which both are resistant to powdery mildew infection and are necrosis-free.

This is achieved by the present invention by providing a powdery mildew-resistant Cucumis sativus plant, as defined in claim 1 . The plant of the invention thus is resistant to powdery mildew and shows no symptoms of leaf necrosis under low-light conditions (i.e. conditions wherein the light exposure of the plants is such that less than 2000 J/cm² of energy is received by the plant = less than 286 J/cm² per day), in particular in combination with a high fruit load.

According to the present invention, a novel necrosis-suppressing genetic factor has been identified. Suitable molecular markers have been developed which can be used to identify and provide Cucumis sativus plants which both are resistant to powdery mildew and are necrosis-free. This novel genetic factor has been found to suppress the powdery mildew-related necrosis. This necrotic suppressing genetic factor is a semi-dominant genetic factor, i.e. both when present in heterozygous and homozygous form, the phenotype will be "necrosis-free".

As demonstrated according to the invention (shown below), the cucumber plant described in EP 1 433 378 does not comprise the necrosis suppressing genetic factor.

In a preferred embodiment of the invention, the plant comprises the known hypocotyl resistance gene (s) and the leaf resistance gene (R) conferring a high level of resistance to the powdery mildew pathogen.

The presence of the powdery mildew resistance genes can be determined using specific molecular markers that are specifically linked to these resistance genes. Suitable markers are known in the art and have for example been described in WO 2007/053015. Thus, as disclosed in WO 2007/053015, the presence of the hypocotyl resistance gene (i.e. the genomic region responsible for the powdery mildew resistance referred to as *pm-h* in WO 2007/053015) is indicated by the presence of specific single nucleotide polymorphism (SNP) markers associated with said powdery mildew resistance gene in said plant. The presence of the leaf resistance gene (i.e. the genomic region responsible for the powdery mildew resistance referred to as *pm-1* in WO 2007/053015) is indicated by the presence of a specific single nucleotide polymorphism (SNP) marker, or a specific insertion mutation marker, indicated as the 5-bp insert 5-AATTT-3". Further markers that may be used to detect the presence of the powdery mildew resistance genes are the AFLP markers E16/M50-F-194, E11/M48-F-251, E23/M38-M001, E23/M40-M003, E24/M46-M002, E24/M46-M003, E12/M48-M003, E26/M43-M003, E14/M59-F-134 and E14/M59-F-200, as described in more detail in WO 2007/053015, to which express reference is made in this context.

According to the invention it has been demonstrated that the necrosis suppressing genetic factor is located on another chromosome as compared to the powdery mildew resistance genes: s and R. This was accomplished by mapping the specific markers for the powdery mildew resistance genes and the necrosis suppressing genetic factor, respectively, at the cucumber chromosomal map.

The necrosis-suppressing genetic factor in the genome of said plant is linked to one or more DNA markers, and can be determined using one or more of said DNA markers. By using DNA markers, plants with the desired combination of powdery mildew resistance and the necrosis-suppressing genetic factor can easily be identified, without the need for performing space and time-consuming necrosis tests. DNA markers may reveal genetic differences that can be visualized by gel electrophoresis and staining with chemicals (e.g. ethidium bromide) or detection with radio-active probes, which are well-known to the person skilled in the art.

The necrosis-suppressing genetic factor is linked to and can be identified by one or more of the DNA markers selected from the group consisting of a first DNA-marker of approximately 65 bp, identified by SEQ ID NO: 1 (GACTGCGTACCAATTCAA) and SEQ ID NO: 2 (GATGAGTCCTGAGTAACCC), and a second DNA-marker of approximately 123 bp, identified by SEQ ID NO: 3 (GACTGCGTACCAATTCAC) and SEQ ID NO: 4 (GATGAGTCCTGAGTAATCG).

According to a preferred embodiment of the present invention, the homozygous presence of the necrosis-suppressing genetic factor in the genome of said plant is identified by the absence of at least one of said DNA markers. Preferably, the homozygous presence of said necrosis-suppressing genetic factor in the genome of said plant is identified by the absence of both the first DNA-marker and the second DNA-marker.

In the research that led to the invention, it has been demonstrated that the absence of said specific molecular marker(s)of the invention in resistant plants is indicative for the necrosis-free fenotype. The molecular markers of the invention thus are a so-called "trans" markers. Homozygous presence of the DNA-fragment (allele) is correlated with the absence of the necrosis suppressing genetic factor and therefore indicative for the non-desired necrotic phenotype. Absence of this DNA-marker thus is indicative for the homozygous presence of the necrosis-suppressing genetic factor, i.e. when the DNA-marker(s) is/are absent, this means that the necrosis-suppressing genetic factor is homozygously present in the genome of the plant.

According to another preferred embodiment of the invention, the heterozygous presence of the necrosis-suppressing genetic factor is identified by the heterozygous presence of the DNA-marker(s). It has been found that the necrotic suppressing genetic factor is a semi-dominant genetic factor, i.e. both when present in homozygous and heterozygous form, the phenotype will be "necrosis-free". Accordingly, the heterozygous presence of the DNA marker(s) according to the invention is indicative for the heterozygous presence of the necrosis-suppressing genetic factor in the plant. Heterozygous presence of the DNA-marker(s) can e.g. be determined using suitable software, such as the AFLP-Quantar®*Pro* developed by Keygene (Wageningen, The Netherlands).

In a particularly preferred embodiment, the plant comprises a necrosis suppressing genetic factor derived from the Cucumis sativus plant, seeds of which have been deposited on 14 February 2006 at the American type culture collection (ATCC), 10801 University Boulevard, Manassas, VA 20110-2209, United States of America under deposit number PTA-7394.

The present invention further relates to the seeds and/or other plant parts of the plants as described above. Plant parts according to the invention are for instance plant cells, pollen, ovules, leaves, embryos, roots, root tips, anthers, flowers, stems, seeds, protoplasts and calli derived from the plant.

In a preferred embodiment, the invention relates to cucumber fruits derived from the plant as described above.

The present invention furthermore relates to a method for obtaining a powdery-mildew resistant Cucumis sativus plant, which is necrosis-free, comprising of introducing a necrosis-suppressing genetic factor into the genome of a powdery mildew-resistant plant.

According to the invention, the powdery mildew resistance genes and the necrosis suppressing genetic factor can be introduced in the genome of the plant using well-known techniques, like classical breeding techniques and/or molecular biological techniques.

According to a preferred embodiment of said method, the powdery mildew resistance genes comprise the known hypocotyl resistance gene (s) and the leaf resistance gene (R). As indicated above, the presence of the powdery mildew resistance genes can be determined using specific markers that are specifically linked to these resistance genes. Suitable markers are known in the art and have for example been described above. The presence of the necrosis suppressing genetic factor is determined using one or more specific DNA markers. The present invention thus provides a simple and reliable method which ensures that the plants of interest can be identified without the need to perform any disease resistance and/or necrosis tests.

The DNA markers for identifying the necrosis-suppressing genetic factor are selected from the group consisting of a first DNA-marker of approximately 65 bp, identified by SEQ ID NO: 1 (GACTGCGTACCAATTCAA) and SEQ ID NO: 2 (GATGAGTCCTGAGTAACCC), and a second DNA-marker of approximately 123 bp, identified by SEQ ID NO: 3 (GACTGCGTACCAATTCAC) and SEQ ID NO: 4 (GATGAGTCCTGAGTAATCG).

In a preferred embodiment, the homozygous presence of the necrosis-suppressing genetic factor is identified by the absence of at least one of said DNA markers. Preferably, the homozygous presence of the necrosis-suppressing genetic factor is identified by the absence of both the first and second DNA markers.

According to another preferred embodiment of the invention, the heterozygous presence of the necrosis-suppressing genetic factor is identified by the heterozygous presence of the DNA-marker(s).

In a particular preferred embodiment, the necrosis suppressing genetic factor is derived from the Cucumis sativus plant of which seeds have been deposited with the ATCC under no. PTA-7394.

The invention further relates to a powdery mildew-resistant Cucumis sativus plant, obtainable by the method as described above, which plant is necrosis-free, as well as to the seeds, and/or other plant parts and fruits of said plant.

In addition, the present invention relates to a method for the identification of necrosis tolerance in a Cucumis sativus plant, comprising detecting the presence of a necrosis-suppressing genetic factor in the genome of said plant using one or more DNA markers, wherein the DNA markers are selected from the group consisting of a first DNA-marker of approximately 65 bp, identified by SEQ ID NO: 1 (GACTGCGTACCAATTCAA) and SEQ ID NO: 2 (GATGAGTCCTGAGTAACCC), and a second DNA-marker of approximately 123 bp, identified by SEQ ID NO: 3 (GACTGCGTACCAATTCAC) and SEQ ID NO: 4 (GATGAGTCCTGAGTAATCG). Using the method of the invention, necrosis-tolerance can easily be detected in Cucumis sativus plants, already in seedlings and/or young plants.

In a preferred embodiment, the homozygous presence of the necrosis-suppressing genetic factor is identified by the absence of at least one of said DNA markers. Preferably, the presence of the necrosis-suppressing genetic factor is identified by the absence of both the first and second DNA markers.

According to another preferred embodiment of the invention, the heterozygous presence of the necrosis-suppressing genetic factor is identified by the heterozygous presence of the DNA-marker(s).

### Explanation of definitions:

The symptoms of powdery mildew resistance can be classified as follows:
According to the present invention, the level of powdery mildew (PM) resistance can be classified as follows:
   level 1 = less than 10% of the surface of first true leaf affected by PM after artificial inoculation, no sporulation, classification: R/resistant;
   level 2 = between 10-50% of surface of first true leave affected by PM after artificial inoculation, some sporulation, classification: IR/intermediate resistant;
   level 3 = more than 50% of the surface of first true leaf affected by PM after artificial inoculation, sporulation, classification: S/susceptible.

According to the present invention, necrosis can be classified as followed:
Level 1: the leaves are green, and the plant is functioning and developing well (classification:
   necrosis-free).
Level 2: yellow spots appear on the leaves, and there is some growth reduction of the leaves
   (classification: intermediate level of necrosis)
Level 3: yellow green leaves with many yellow spots, very serious growth problems, ultimately resulting in partially or complete dying leaves (necrosis) and sometimes even death of the plant (classification:
   necrosis).

The wording "low light conditions" relate to i.e. conditions wherein the light exposure of the plants is such that less than 2000 J/cm² of energy is received by the plant = less than 286 J/cm² per day. Under these conditions, symptoms of necrosis will occur in plants that do not comprise the necrosis-suppressing genetic factor of the invention.

A high fruit load according to the invention relates to a fruit load of at least one fully developed (i.e. in a harvestable stage) fruit per node.

The term "necrosis-suppressing genetic factor" as used according to the present invention relates to a DNA fragment determining and transmitting the necrosis-suppressing property from parent to offspring. It has been found according to the invention that the necrosis-free genetic factor is semi-dominant, i.e. both when present homozygously and heterozygously, the necrosis-free phenotype is observed.

A DNA marker according to the invention refers to a DNA sequence that can be identified by a simple assay, e.g. PCR followed by electrophoresis, allowing the presence or absence of neighbouring stretches of the genome to be inferred. The marker may e.g. be an AFLP marker.

The present invention is further illustrated by the following Example.

### EXAMPLES

In the research that led to the present invention a novel necrosis-reducing factor has been identified in Cucumis sativus plants.

A segregating population of a powdery mildew hypocotyl and leaf resistant, necrotic Cucumis sativus (Code B, see table 1) X a powdery mildew hypocotyl and leaf resistant, necrosis-free Cucumis sativus (Code A, deposited at 14 February 2006 with the ATCC under number PTA-7394) was produced. AFLP-markers linked to the necrosis-suppressing genetic factor were identified using a Bulked Segregant Analysis (BSA) approach (Michelmore et al., PNAS 88:9828-98232, 1991). Markers linked to the necrosis-suppressing factor could be mapped on a linkage group which is distinct from the linkage group which is harboring the powdery mildew resistance genes.

Validation of the markers linked to the necrosis-suppressing genetic factor was performed by screening these markers on plants of the segregating population and a specific panel of breeding lines, according to well-known molecular biological methods.

The molecular markers described in WO 2007/053015 and identified in table 3, were used to determine the presence/absence of the powdery mildew resistance genes.

**Table 1. Necrosis suppressing genetic factor Marker results with different genotypes**

| Genotype | PM-resistance level | Necrosis level | Marker 65 bp | Marker 123 bp |
|---|---|---|---|---|
| Code A | 1 | 1 | - | - |
| Code B | 1 | 3 | + homozygous | + homozygous |
| cv Flamingo F1 * | 2 | 2 | + homozygous | + homozygous |

| | | | | |
|---|---|---|---|---|
| + = marker is present - = marker is absent * = plant according to EP 1 433 378 the scores 1-3 are explained above. | | | | |

It thus becomes clear that in the plant according to the invention (Code A), both of the DNA markers, that have been identified as being linked to the novel necrosis suppressing genetic factor of the invention, are absent, indicating the presence of the necrosis suppressing genetic factor and thus of the necrosis-free fenotype. In contrast, in the plant indicated by Code B (resistant, necrotic) and in the plant described in EP 1 433 378, referred to above, both of these DNA markers are present, indicating that these plants do not comprise the necrosis suppressing genetic factor of the present invention.

The presence of the powdery mildew resistance genes of these plants has also been tested using the molecular markers (listed in table 3). The results of both markers analyses have been summarized in table 2.

The results clearly show that the plants identified by Code A and Code B (genotypes A en B) score homozygous for all powdery mildew markers and show the highest level of powdery mildew resistance, whereas cv. Flamingo (i.e. the plant of EP 1 433 378) scores heterozygous for the PM markers identified by SEQ ID NO: 7 and 8 and shows a lower level of powdery mildew resistance.

These marker data thus show the independent segregation behaviour of the powdery mildew resistance markers relative to the necrosis markers. This clearly demonstrates that the powdery mildew resistance and the necrosis suppressing genetic factor are unlinked.

### Combined PM/necrosis seedling test protocol:

### Plant material

The time to perform the experiment in the Netherlands is from 1 November until 1 February (low light conditions, < 2000 J/cm² of energy per week = 286 J/cm² per day). Seedlings (test plants and controls) are grown at 24 ° C in vermiculite covered with sand. The seedlings are transplanted in a ground table after 4 to 5 days (cotyledons just spread). Controls are necrosis-susceptible, PM-resistant plants.

### Pathogen

Sphaerotheca fuliginea (Podosphaera xanthii) race 2 multiplied on Kamaron, a commercially available F1 hybrid.

**Table 2 Powdery mildew resistance Marker results**

| Genotype | PM resistance level | Necrosis level | Marker SEQ ID NO: 5 | Marker SEQ ID NO: 6 | Marker SEQ ID NO: 7 | Marker SEQ ID NO: 8 | Marker 65 bp | Marker 123 bp |
|---|---|---|---|---|---|---|---|---|
| Code A 1 | | 1 | + homozygous | + homozygous | + homozygous | + homozygous | - | - |
| Code B | 1 | 3 | + homozygous | + homozygous | + homozygous | + homozygous | + homozygous | + homozygous |
| cv. Flamingo | 2 | 2 | + homozygous | + homozygous | + *heterozygous* | + *heterozygous* | + homozygous | + homozygous |

### Preparation of inoculum

Well sporulating leaves are taken and the spores rubbed off into water; the inoculum is sieved by pouring the inoculum through a funnel covered with thoroughly wetted cheesecloth.

The viability of the spores is checked by using an UV-microscope after staining with FDA (fluorescein diacetate) and, after counting, the concentration of viable spores is adjusted to approximately 1 X 10⁵ viable spores/ml for the first inoculation on hypocotyls, and approximately 5 x 10⁴ viable spores for the second inoculation on the first leaf.

### Inoculation

The seedlings are inoculated (with a sprayer) 1-2 days after transplanting. A second infection is made when the first leaf has just spread (4 to 6 days a.t.).

The humidity can be increased by wetting the soil directly after inoculation to stimulate infection. Temperature at night: 18-20° C, in the daytime: 22-25° C.

### Growth measurement

In case of low humidity after 5 days (after infection), the sporulation can be stimulated by wetting the soil once or twice every day.

### Development of symptoms

The necrosis in young plants is scored approximately 14 days after the last inoculation. The scores of necrosis are determined as identified above. The powdery mildew infection on hypocotyl and leaves is also scored approximately 14 days after the last inoculation. The scores of mildew infection are determined as identified above.

**Table 3 Marker sequences powdery mildew**

| |
|---|
| >PMHypocotyl1- SEQ ID NO: 5 TCATAATGACACGTAATGATTGTCAGAGRAAATTTATAGAAACCTTTTGTTCAA CTATCCAACAAATTACAATCAAGGCACTTCTGGAATGAGATAGTCA |
| >PMHypocotyl2- SEQ ID NO: 6 GTCGTCTTCGCCTATGCaAGACAAAATAAATGCTTGTTTKAGTCTAGCCAAAAA TGGTGTAGAACAGTTGATCACAGTTCCTACGGACTA |
| >PM-Leafl- SEQ ID NO: 7 TGGATAAGAGAGGTYCTTGTAAAATRTTATTTTCATTTAGACCTTGATtttaaTTT GGACTATGAATCATATTTGACAATTGTAGGATCAAACCGAAGGTGCA |
| >PM-Leaf 2- SEQ ID NO: 8 GAGAGGATTCATRTTCATCTTCTCCCAGGTGCTACAATCGAAAGAATTYATCTT CATCTTCTCTTAGGTGCCACAATCGAGAGGGTTTATCTTCATCTTTC |

## Claims

1. Powdery mildew-resistant *Cucumis sativus* plant, comprising in its genome a necrosis-suppressing genetic factor, which plant is resistant to powdery mildew and necrosis-free under conditions wherein the light exposure of the plant is less than 2000 J/cm², and wherein the presence of the necrosis-suppressing genetic factor is determinable by the absence of a first DNA-marker of approximately 65 bp, identified by SEQ ID NO: 1 (GACTGCGTACCAATTCAA) and SEQ ID NO: 2 (GATGAGTCCTGAGTAACCC), and the absence of a second DNA-marker of approximately 123 bp, identified by SEQ ID NO: 3 (GACTGCGTACCAATTCAC) and SEQ ID NO: 4 (GATGAGTCCTGAGTAATCG).

2. Plant according to claim 1, wherein the plant comprises the hypocotyl resistance gene (s) and the leaf resistance gene (R).

3. Plant according to claim 1 or 2, wherein the presence of the powdery mildew resistance gene is determinable by one or more specific markers selected from the group consisting of the markers comprising a nucleotide sequence identified by SEQ ID NO: 5-8 in Table 3, and the AFLP markers E16/M50-F-194, E11/M48-F-251, E23/M38-M001, E23/M40-M003, E24/M46-M002, E24/M46-M003, E12/M48-M003, E26/M43-M003, E14/M59-F-134 and E14/M59-F-200.

4. Plant according to any one of the preceding claims 1 to 3 comprising in its genome a necrosis suppressing genetic factor derived from the *Cucumis sativus* plant, of which seeds have been deposited with the American Type Culture Collection (ATCC) on 14 February 2006 under deposit number PTA-7394.

5. Seeds and/or other plant parts of a plant according to any one of the claims 1 to 4, said seeds and/or other plant parts comprise in their genome a necrosis-suppressing genetic factor, and which plant thereof are resistant to powdery mildew and necrosis-free under conditions wherein the light exposure of the plant is less than 2000 J/cm², and wherein the presence of the necrosis-suppressing genetic factor is determinable by the absence of a first DNA-marker of approximately 65 bp, identified by SEQ ID NO: 1 (GACTGCGTACCAATTCAA) and SEQ ID NO: 2 (GATGAGTCCTGAGTAACCC), and the absence of a second DNA-marker of approximately 123 bp, identified by SEQ ID NO: 3 (GACTGCGTACCAATTCAC) and SEQ ID NO: 4 (GATGAGTCCTGAGTAATCG).

6. Cucumber fruits derived from a plant according to any of the claims 1 to 4, said cucumber fruits comprise in their genome a necrosis-suppressing genetic factor, wherein the presence of the necrosis-suppressing genetic factor is determinable by the absence of a first DNA-marker of approximately 65 bp, identified by SEQ ID NO: 1 (GACTGCGTACCAATTCAA) and SEQ ID NO: 2 (GATGAGTCCTGAGTAACCC), and the absence of a second DNA-marker of approximately 123 bp, identified by SEQ ID NO: 3 (GACTGCGTACCAATTCAC) and SEQ ID NO: 4 (GATGAGTCCTGAGTAATCG).

7. Method for obtaining a *Cucumis sativus plant,* which plant is resistant to powdery mildew and necrosis-free under conditions wherein the light exposure of the plant is less than 2000 J/cm², comprising introducing a necrosis-suppressing genetic factor into the genome of a powdery mildew resistant plant, wherein the presence of the necrosis-suppressing genetic factor is determinable by the absence of a first DNA-marker of approximately 65 bp, identified by SEQ ID NO: 1 (GACTGCGTACCAATTCAA) and SEQ ID NO: 2 (GATGAGTCCTGAGTAACCC), and the absence of a second DNA-marker of approximately 123 bp, identified by SEQ ID NO: 3 (GACTGCGTACCAATTCAC) and SEQ ID NO: 4 (GATGAGTCCTGAGTAATCG).

8. Method according to claim 7, wherein the powdery mildew resistance genes comprise the hypocotyl resistance gene (s) and the leaf resistance gene (R).

9. Method according to claim 7 or 8, wherein the presence of the powdery mildew resistance genes is determined by one or more specific markers selected from the group consisting of the markers comprising a nucleotide sequence identified by SEQ ID NO: 5-8, and the AFLP markers E16/M50-F-194, E11/M48-F-251, E23/M38-M001, E23/M40-M003, E24/M46-M002, E24/M46-M003, E12/M48-M003, E26/M43-M003, E14/M59-F-134 and E14/M59-F-200.

10. Method according to any of the claims 7 to 9, wherein the necrosis-suppressing genetic factor has been derived from the *Cucumis sativus* plant, of which seeds have been deposited with the ATCC, under no. PTA-7394.

11. *Cucumis sativus* plant obtainable by a method according to any of the claims 7 to 10, which plant is resistant to powdery mildew and necrosis free under conditions wherein the light exposure of the plant is less than 2000 J/cm².

12. Seeds and/or other plant parts of the plant according to claim 11 said seeds and/or other plant part comprise in their genome a necrosis-suppressing genetic factor, which plant thereof are resistant to powdery mildew and necrosis-free under conditions wherein the light exposure of the plant is less than 2000 J/cm², and wherein the presence of the necrosis-suppressing genetic factor is determinable by the absence of a first DNA-marker of approximately 65 bp, identified by SEQ ID NO: 1 (GACTGCGTACCAATTCAA) and SEQ ID NO: 2 (GATGAGTCCTGAGTAACCC), and the absence of a second DNA-marker of approximately 123 bp, identified by SEQ ID NO: 3 (GACTGCGTACCAATTCAC) and SEQ ID NO: 4 (GATGAGTCCTGAGTAATCG).

13. Cucumber fruits derived from a plant according to claim 11 said cucumber fruits comprise in their genome a necrosis-suppressing genetic factor, wherein the presence of the necrosis-suppressing genetic factor is determinable by the absence of a first DNA-marker of approximately 65 bp, identified by SEQ ID NO: 1 (GACTGCGTACCAATTCAA) and SEQ ID NO: 2 (GATGAGTCCTGAGTAACCC), and the absence of a second DNA-marker of approximately 123 bp, identified by SEQ ID NO: 3 (GACTGCGTACCAATTCAC) and SEQ ID NO: 4 (GATGAGTCCTGAGTAATCG).

14. Method for identification of necrosis tolerance in a *Cucumis sativus plant*, comprising detecting the presence of a necrosis-suppressing genetic factor in the genome of said plant using the absence of a first DNA-marker of approximately 65 bp, identified by SEQ ID NO: 1 (GACTGCGTACCAATTCAA) and SEQ ID NO: 2 (GATGAGTCCTGAGTAACCC), and a second DNA-marker of approximately 123 bp, identified by SEQ ID NO: 3 (GACTGCGTACCAATTCAC) and SEQ ID NO: 4 (GATGAGTCCTGAGTAATCG).

## Patentansprüche

1. Gegen echten Mehltau resistente *Cucumis sativus-*Pflanze, die in ihrem Genom einen Nekrose unterdrückenden genetischen Faktor umfasst, wobei die Pflanze unter Bedingungen gegen echten Mehltau resistent und frei von Nekrose ist, bei welchen die Lichtexposition der Pflanze weniger als 2000 J/cm² beträgt, und wobei das Vorliegen des Nekrose unterdrückenden genetischen Faktors durch das Fehlen eines ersten DNA-Markers von ungefähr 65 bp, der durch SEQ ID NR: 1 (GACTGCGTACCAATTCAA) und SEQ ID NR: 2 (GATGAGTCCTGAGTAACCC) identifiziert wird, und das Fehlen eines zweiten DNA-Markers von ungefähr 123 bp, der durch SEQ ID NR: 3 (GACTGCGTACCAATTCAC) und SEQ ID NR: 4 (GATGAGTCCTGAGTAATCG) identifiziert wird, bestimmbar ist.

2. Pflanze gemäß Anspruch 1, wobei die Pflanze das Hypocotyl-Resistenzgen (s) und das Blatt-Resistenzgen (R) umfasst.

3. Pflanze gemäß Anspruch 1 oder 2, wobei das Vorliegen des Resistenzgens gegen echten Mehltau durch einen oder mehrere spezifische Marker bestimmbar ist, die ausgewählt sind aus der Gruppe, die aus den Markern, welche eine Nukleotidsequenz umfassen, die durch SEQ ID NR: 5 - 8 in Tabelle 3 identifiziert wird, und den AFLP-Markern E16/M50-F-194, E11/M48-F-251, E23/M38-M001, E23/M40-M003, E24/M46-M002, E24/M46-M003, E12/M48-M003, E26/M43-M003, E14/M59-F-134 und E14/M59-F-200 besteht.

4. Pflanze gemäß irgendeinem der vorhergehenden Ansprüche 1 bis 3, die in ihrem Genom einen Nekrose unterdrückenden genetischen Faktor umfasst, der von der *Cucumis sativus*-Pflanze abgeleitet ist, von welcher Samen am 14. Februar 2006 unter der Hinterlegungsnummer PTA-7394 bei der American Type Culture Collection (ATCC) hinterlegt wurden.

5. Samen und/oder andere Pflanzenteile einer Pflanze gemäß irgendeinem der Ansprüche 1 bis 4, wobei die Samen und/oder anderen Pflanzenteile in ihrem Genom einen Nekrose unterdrückenden genetischen Faktor umfassen, und wobei die Pflanze daraus unter Bedingungen gegen echten Mehltau resistent und frei von Nekrose ist, bei welchen die Lichtexposition der Pflanze weniger als 2000 J/cm² beträgt, und wobei das Vorliegen des Nekrose unterdrückenden genetischen Faktors durch das Fehlen eines ersten DNA-Markers von ungefähr 65 bp, der durch SEQ ID NR: 1 (GACTGCGTACCAATTCAA) und SEQ ID NR: 2 (GATGAGTCCTGAGTAACCC) identifiziert wird, und das Fehlen eines zweiten DNA-Markers von ungefähr 123 bp, der durch SEQ ID NR: 3 (GACTGCGTACCAATTCAC) und SEQ ID NR: 4 (GATGAGTCCTGAGTAATCG) identifiziert wird, bestimmbar ist.

6. Gurkenfrüchte, die von einer Pflanze gemäß irgendeinem der Ansprüche 1 bis 4 abgeleitet sind, wobei die Gurkenfrüchte in ihrem Genom einen Nekrose unterdrückenden genetischen Faktor umfassen, wobei das Vorliegen des Nekrose unterdrückenden genetischen Faktors durch das Fehlen eines ersten DNA-Markers von ungefähr 65 bp, der durch SEQ ID NR: 1 (GACTGCGTACCAATTCAA) und SEQ ID NR: 2 (GATGAGTCCTGAGTAACCC) identifiziert wird, und das Fehlen eines zweiten DNA-Markers von ungefähr 123 bp, der durch SEQ ID NR: 3 (GACTGCGTACCAATTCAC) und SEQ ID NR: 4 (GATGAGTCCTGAGTAATCG) identifiziert wird, bestimmbar ist.

7. Verfahren zum Erhalt einer *Cucumis sativus-*Pflanze, wobei die Pflanze unter Bedingungen gegen echten Mehltau resistent und frei von Nekrose ist, bei welchen die Lichtexposition der Pflanze weniger als 2000 J/cm² beträgt, welches das Einführen eines Nekrose unterdrückenden genetischen Faktors in das Genom einer gegen echten Mehltau resistenten Pflanze umfasst, wobei das Vorliegen des Nekrose unterdrückenden genetischen Faktors durch das Fehlen eines ersten DNA-Markers von ungefähr 65 bp, der durch SEQ ID NR: 1 (GACTGCGTACCAATTCAA) und SEQ ID NR: 2 (GATGAGTCCTGAGTAACCC) identifiziert wird, und das Fehlen eines zweiten DNA-Markers von ungefähr 123 bp, der durch SEQ ID NR: 3 (GACTGCGTACCAATTCAC) und SEQ ID NR: 4 (GATGAGTCCTGAGTAATCG) identifiziert wird, bestimmbar ist.

8. Verfahren gemäß Anspruch 7, wobei die Resistenzgene gegen echten Mehltau das Hypocotyl-Resistenzgen (s) und das Blatt-Resistenzgen (R) umfassen.

9. Verfahren gemäß Anspruch 7 oder 8, wobei das Vorliegen der Resistenzgene gegen echten Mehltau durch einen oder mehrere spezifische Marker bestimmbar ist, die ausgewählt sind aus der Gruppe, die aus den Markern, welche eine Nukleotidsequenz umfassen, die durch SEQ ID NR: 5 - 8 identifiziert wird, und den AFLP-Markern E16/M50-F-194, E11/M48-F-251, E23/M38-M001, E23/M40-M003, E24/M46-M002, E24/M46-M003, E12/M48-M003, E26/M43-M003, E14/M59-F-134 und E14/M59-F-200 besteht.

10. Verfahren gemäß irgendeinem der Ansprüche 7 bis 9, wobei der Nekrose unterdrückende genetische Faktor von der *Cucumis sativus*-Pflanze abgeleitet wurde, von welcher Samen bei der ATCC unter der Nr. PTA-7394 hinterlegt wurden.

11. *Cucumis sativus*-Pflanze, die durch ein Verfahren gemäß irgendeinem der Ansprüche 7 bis 10 erhältlich ist, wobei die Pflanze unter Bedingungen gegen echten Mehltau resistent und frei von Nekrose ist, bei welchen die Lichtexposition der Pflanze weniger als 2000 J/cm² beträgt.

12. Samen und/oder andere Pflanzenteile der Pflanze gemäß Anspruch 11, wobei die Samen und/oder anderen Pflanzenteile in ihrem Genom einen Nekrose unterdrückenden genetischen Faktor umfassen, wobei die Pflanze daraus unter Bedingungen gegen echten Mehltau resistent und frei von Nekrose ist, bei welchen die Lichtexposition der Pflanze weniger als 2000 J/cm² beträgt, und wobei das Vorliegen des Nekrose unterdrückenden genetischen Faktors durch das Fehlen eines ersten DNA-Markers von ungefähr 65 bp, der durch SEQ ID NR: 1 (GACTGCGTACCAATTCAA) und SEQ ID NR: 2 (GATGAGTCCTGAGTAACCC) identifiziert wird, und das Fehlen eines zweiten DNA-Markers von ungefähr 123 bp, der durch SEQ ID NR: 3 (GACTGCGTACCAATTCAC) und SEQ ID NR: 4 (GATGAGTCCTGAGTAATCG) identifiziert wird, bestimmbar ist.

13. Gurkenfrüchte, die von einer Pflanze gemäß Anspruch 11 abgeleitet sind, wobei die Gurkenfrüchte in ihrem Genom einen Nekrose unterdrückenden genetischen Faktor umfassen, wobei das Vorliegen des Nekrose unterdrückenden genetischen Faktors durch das Fehlen eines ersten DNA-Markers von ungefähr 65 bp, der durch SEQ ID NR: 1 (GACTGCGTACCAATTCAA) und SEQ ID NR: 2 (GATGAGTCCTGAGTAACCC) identifiziert wird, und das Fehlen eines zweiten DNA-Markers von ungefähr 123 bp, der durch SEQ ID NR: 3 (GACTGCGTACCAATTCAC) und SEQ ID NR: 4 (GATGAGTCCTGAGTAATCG) identifiziert wird, bestimmbar ist.

14. Verfahren zur Feststellung der Nekrosetoleranz in einer *Cucumis sativus*-Pflanze, welches das Nachweisen des Vorliegens eines Nekrose unterdrückenden genetischen Faktors in dem Genom der Pflanze umfasst, wobei das Fehlen eines ersten DNA-Markers von ungefähr 65 bp, der durch SEQ ID NR: 1 (GACTGCGTACCAATTCAA) und SEQ ID NR: 2 (GATGAGTCCTGAGTAACCC) identifiziert wird, und eines zweiten DNA-Markers von ungefähr 123 bp, der durch SEQ ID NR: 3 (GACTGCGTACCAATTCAC) und SEQ ID NR: 4 (GATGAGTCCTGAGTAATCG) identifiziert wird, verwendet wird.

## Revendications

1. Plante de *Cucumis sativus* résistante à l'oïdium, comprenant dans son génome un facteur génétique supprimant la nécrose, laquelle plante est résistante à l'oïdium et dépourvue de nécrose dans des conditions dans lesquelles l'exposition de la plante à la lumière est inférieure à 2 000 J/cm², et où la présence du facteur génétique supprimant la nécrose peut être déterminée par l'absence d'un premier marqueur d'ADN d'approximativement 65 pb, identifié par SEQ ID NO: 1 (GACTGCGTACCAATTCAA) et SEQ ID NO: 2 (GATGAGTCCTGAGTAACCC), et l'absence d'un second marqueur d'ADN d'approximativement 123 pb, identifié par SEQ ID NO: 3 (GACTGCGTACCAATTCAC) et SEQ ID NO: 4 (GATGAGTCCTGAGTAATCG).

2. Plante selon la revendication 1, dans laquelle la plante comprend le gène de résistance d'hypocotyle (s) et le gène de résistance de feuille (R).

3. Plante selon la revendication 1 ou 2, dans laquelle la présence du gène de résistance à l'oïdium peut être déterminée par un ou plusieurs marqueurs spécifiques choisis dans le groupe consistant en les marqueurs comprenant une séquence de nucléotides identifiée par SEQ ID NO: 5-8 dans le tableau 3, et les marqueurs AFLP E16/M50-F-194, E11/M48-F-251, E23/M38-M001, E23/M40-M003, E24/M46-M002, E24/M46-M003, E12/M48-M003, E26/M43-M003, E14/M59-F-134 et E14/M59-F-200.

4. Plante selon l'une quelconque des revendications 1 à 3 précédentes, comprenant dans son génome un facteur génétique supprimant la nécrose dérivé de la plante de *Cucumis sativus*, dont les graines ont été déposées auprès de l'American Type Culture Collection (ATCC) le 14 février 2006 sous le numéro de dépôt PTA-7394.

5. Graines et/ou autres parties de plante d'une plante selon l'une quelconque des revendications 1 à 4, lesdites graines et/ou autres parties de plante comprenant dans leur génome un facteur génétique supprimant la nécrose, et dont les plantes sont résistantes à l'oïdium et dépourvues de nécrose dans des conditions dans lesquelles l'exposition de la plante à la lumière est inférieure à 2 000 J/cm², et où la présence du facteur génétique supprimant la nécrose peut être déterminée par l'absence d'un premier marqueur d'ADN d'approximativement 65 pb, identifié par SEQ ID NO: 1 (GACTGCGTACCAATTCAA) et SEQ ID NO: 2 (GATGAGTCCTGAGTAACCC), et l'absence d'un second marqueur d'ADN d'approximativement 123 pb, identifié par SEQ ID NO: 3 (GACTGCGTACCAATTCAC) et SEQ ID NO: 4 (GATGAGTCCTGAGTAATCG).

6. Fruits de concombre dérivés d'une plante selon l'une quelconque des revendications 1 à 4, lesdits fruits de concombre comprenant dans leur génome un facteur génétique supprimant la nécrose, où la présence du facteur génétique supprimant la nécrose peut être déterminée par l'absence d'un premier marqueur d'ADN d'approximativement 65 pb, identifié par SEQ ID NO: 1 (GACTGCGTACCAATTCAA) et SEQ ID NO: 2 (GATGAGTCCTGAGTAACCC), et l'absence d'un second marqueur d'ADN d'approximativement 123 pb, identifié par SEQ ID NO: 3 (GACTGCGTACCAATTCAC) et SEQ ID NO: 4 (GATGAGTCCTGAGTAATCG).

7. Procédé pour obtenir une plante de *Cucumis sativus*, laquelle plante est résistante à l'oïdium est dépourvue de nécrose dans des conditions dans lesquelles l'exposition de la plante à la lumière est inférieure à 2 000 J/cm², comportant l'introduction d'un facteur génétique supprimant la nécrose dans le génome d'une plante résistante à l'oïdium, où la présence du facteur génétique supprimant la nécrose peut être déterminée par l'absence d'un premier marqueur d'ADN d'approximativement 65 pb, identifié par SEQ ID NO: 1 (GACTGCGTACCAATTCAA) et SEQ ID NO: 2 (GATGAGTCCTGAGTAACCC), et l'absence d'un second marqueur d'ADN d'approximativement 123 pb, identifié par SEQ ID NO: 3 (GACTGCGTACCAATTCAC) et SEQ ID NO: 4 (GATGAGTCCTGAGTAATCG).

8. Procédé selon la revendication 7, dans lequel les gènes de résistance à l'oïdium comprennent le gène de résistance d'hypocotyle (s) et le gène de résistance de feuille (R).

9. Procédé selon la revendication 7 ou 8, dans lequel la présence des gènes de résistance à l'oïdium est déterminée par un ou plusieurs marqueurs spécifiques choisis dans le groupe consistant en les marqueurs comprenant une séquence de nucléotides identifiée par SEQ ID NO: 5-8, et les marqueurs AFLP E16/M50-F-194, E11/M48-F-251, E23/M38-M001, E23/M40-M003, E24/M46-M002, E24/M46-M003, E12/M48-M003, E26/M43-M003, E14/M59-F-134 et E14/M59-F-200.

10. Procédé selon l'une quelconque des revendications 7 à 9, dans lequel le facteur génétique supprimant la nécrose a été dérivé de la plante de *Cucumis sativus*, dont les graines ont été déposées auprès de l'ATCC, sous le numéro PTA-7394.

11. Plante de *Cucumis sativus* pouvant être obtenue par un procédé selon l'une quelconque des revendications 7 à 10, laquelle plante est résistante à l'oïdium et dépourvue de nécrose dans des conditions dans lesquelles l'exposition de la plante à la lumière est inférieure à 2 000 J/cm².

12. Graines et/ou autres parties de plante de la plante selon la revendication 11, lesdites graines et/ou autres parties de plante comprenant dans leur génome un facteur génétique supprimant la nécrose, laquelle plante est résistante à l'oïdium et dépourvue de nécrose dans des conditions dans lesquelles l'exposition de la plante à la lumière est inférieure à 2 000 J/cm², et où la présence du facteur génétique supprimant la nécrose peut être déterminée par l'absence d'un premier marqueur d'ADN d'approximativement 65 pb, identifié par SEQ ID NO: 1 (GACTGCGTACCAATTCAA) et SEQ ID NO: 2 (GATGAGTCCTGAGTAACCC), et l'absence d'un second marqueur d'ADN d'approximativement 123 pb, identifié par SEQ ID NO: 3 (GACTGCGTACCAATTCAC) et SEQ ID NO: 4 (GATGAGTCCTGAGTAATCG).

13. Fruits de concombre dérivés d'une plante selon la revendication 11, lesdits fruits de concombre comprenant dans leur génome un facteur génétique supprimant la nécrose, où la présence du facteur génétique supprimant la nécrose peut être déterminée par l'absence d'un premier marqueur d'ADN d'approximativement 65 pb, identifié par SEQ ID NO: 1 (GACTGCGTACCAATTCAA) et SEQ ID NO: 2 (GATGAGTCCTGAGTAACCC), et l'absence d'un second marqueur d'ADN d'approximativement 123 pb, identifié par SEQ ID NO: 3 (GACTGCGTACCAATTCAC) et SEQ ID NO: 4 (GATGAGTCCTGAGTAATCG).

14. Procédé d'identification d'une tolérance à la nécrose dans une plante de *Cucumis sativus*, comprenant la détection de la présence d'un facteur génétique supprimant la nécrose dans le génome de ladite plante au moyen de l'absence d'un premier marqueur d'ADN d'approximativement 65 pb, identifié par SEQ ID NO: 1 (GACTGCGTACCAATTCAA) et SEQ ID NO: 2 (GATGAGTCCTGAGTAACCC), et d'un second marqueur d'ADN d'approximativement 123 pb, identifié par SEQ ID NO: 3 (GACTGCGTACCAATTCAC) et SEQ ID NO: 4 (GATGAGTCCTGAGTAATCG).
